# EUROPÄISCHE PATENTANMELDUNG

(11) **EP 0 531 700 A2**
(43) Veröffentlichungstag der Anmeldung: **17.03.1993**
(21) Anmeldenummer: 92113174.4
(22) Anmeldetag: 03.08.1992
(51) Int. Cl.: C07C 69/738, C07C 67/46, C07C 51/09, C07C 59/84, C07C 45/65, C07C 49/88, C07C 51/493

(54) **Diastereomerenreine Zwischenprodukte und ihre Verwendung bei der Herstellung von (R)- oder (S)-Ketoprofen**

(30) Priorität: 14.08.1991 DE 4126859
(71) Anmelder: BAYER AG, 51368 Leverkusen (DE)
(72) Erfinder: Schröder, Georg, Dr., W-5090 Leverkusen 1 (DE); Arlt, Dieter, Prof. Dr., W-5000 Köln 80 (DE)

(57) **Zusammenfassung**

Die Erfindung betrifft neue diastereomerenreine Ester des Ketoprofens, Verfahren Zu ihrer Herstellung durch asymmetrische Keten-Addition und ihre Verwendung bei der Herstellung von (R)- oder (S)-Ketoprofen.

## Beschreibung

Die Erfindung betrifft neue diastereomerenreine Ester des Ketoprofens, Verfahren zu ihrer Herstellung durch asymmetrische Keten-Addition und ihre Verwendung bei der Herstellung von (R)- oder (S)-Ketoprofen.

Pharmakologische Eigenschaften von Wirkstoffen hängen sehr oft von der absoluten Stereochemie ab. Dieses Phänomen wurde auch bei nichtsteroidalen Analgetika und Antipyretika aus der Klasse der 2-Arylpropionsäuren beobachtet. Aus diesen Gründen sind verschiedene Methoden zur Herstellung enantiomerenreiner Carbonsäuren mit chiralem α-C-Atom ausgearbeitet worden.

Die Herstellung von (S)- oder (R)-Ketoprofen gelingt auf bisher bekannten Wegen nur sehr schlecht. Bei der Racematspaltung von 2-(3-Benzylphenyl)-propionsäure und auch bei der asymmetrischen Hydrierung von Dehydroketoprofen mit chiralen Rhodium-Katalysatoren, erhält man nur schlechte Ausbeuten ebenso wie nach Oxidation zu (S)-Ketoprofen (vgl. G. Comisso et al., Gazz. Chim. Italia 110, 123 (1980)). Dies gilt auch für die Racematspaltung von 2-(2-Thiaxanthonyl)-propionsäuren mit nachfolgender mehrstufiger Überführung in (S)-Ketoprofen (vgl. US 3 641 127) und für die direkte Racematspaltung mit Phenethylamin (vgl. DE 3 824 353). Auch durch chromatographische Diastereomerentrennung der Amide des Ketoprofens mit chiralen Aminen sind nach Verseifung der Amide nur kleine Mengen von (S)-Ketoprofen zugänglich (vgl. Abas et al., J. Pharmacol. Exp. Ther. 240, 637 (1987)). Auch enzymatische Esterhydrolysen versagen bei der Herstellung von enantiomerenreinem Ketoprofen (vgl. S. H. Wu, et al., J. Am. Chem. Soc., 112, 1990 (1990)).

Zusammenfassend kann gesagt werden, daß bisher für die technische Produktion größerer Mengen von (S)- oder (R)-Ketoprofen keine geeigneten Herstellungsverfahren bekannt sind.

Es ist weiterhin bekannt, aktivierte racemische Carbonsäurederivate unter Katalyse mit chiralen Titankomplexen zu enantiomer angereicherten Estern umzusetzen (vgl. K. Narasaka et al., Chem. Lett. 1989, 1187). Diese Reaktionen haben gegenüber der klassischen Racematspaltung den Vorteil, daß sie prinzipiell größere Ausbeuten als 50 % liefern können. Aus US 4 691 020 und EP 390 273 sind weiterhin Additionen von optisch aktiven Alkoholen und α-Hydroxyestern oder α-Hydroxyamiden an bestimmte Ketene bekannt, die zu Estern mit Diastereomerenüberschüssen führen.

Es zeigt sich, daß sich die bisher bekannten und üblichen Methoden zur Auftrennung von optischen Isomeren von 2-Arylpropionsäuren nicht ohne weiteres auf Ketoprofen übertragen lassen, Methoden die z.B. bei der Arylpropionsäure Ibuprofen erfolgreich eingesetzt werden können, versagen bei ihrer Übertragung auf Ketoprofen oder liefern nur unzureichende Ausbeuten. Bei Vergleich der Strukturen konnte der Fachmann davon ausgehen, daß beim Ketoprofen besonders schwierige sterische Verhältnisse vorliegen.

Überraschenderweise wurde gefunden, daß man (S)- bzw. (R)-Ketoprofen der Formeln Ia und Ib 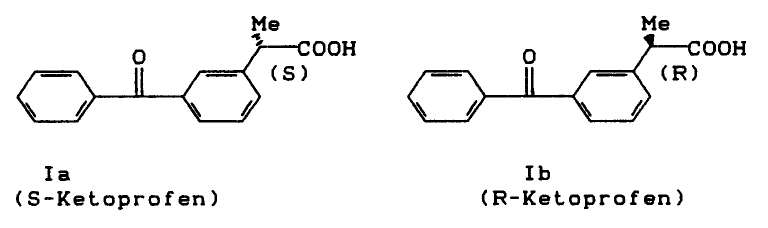
in einfacher Weise, in hoher Reinheit und in guten Ausbeuten erhält, wenn man racemisches Ketoprofen der Formel 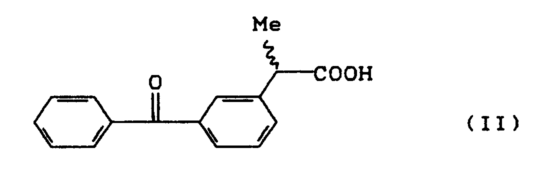
über das Säurechlorid in Anwesenheit von tertiären Aminen zu Ketenen der allgemeinen Formel (III) 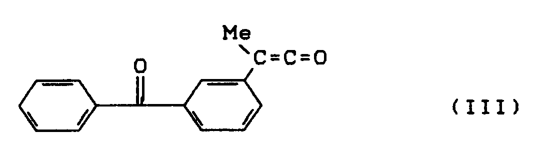
umsetzt, dieses Keten dann in ein separates Reaktionsgefäß überführt und dort bei Temperaturen zwischen -100°C und +25°C mit Alkoholen der allgemeinen Formel (IV)

R¹-OH (IV)

in welcher
- R¹: für den Rest 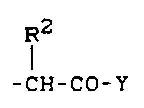 steht, wobei
- R²: für einen C₁₋₁₀-Alkylrest, C₃₋₅-Cycloalkylrest, Benzyl oder Phenyl steht, und
- Y: für einen C₁₋₁₀-Alkoxyrest, einen C₃₋₁₀-Cycloalkyloxyrest, Benzyloxy oder Phenoxy steht,
oder
- Y: für einen Aminrest steht, der durch Alkyl oder Cycloalkyl mit bis zu 10 C-Atomen, Benzyl oder Phenyl gleich oder verschieden substituiert ist,
oder
- R¹: für einen Rest des optisch aktiven α-Hydroxylactons oder oder eines cyclischen Amids gemäß der Formel 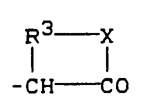 steht, worin
- R³: für einen C₂₋₄-Alkylenrest steht, der gegebenenfalls durch 1 oder 2 C₁₋₁₀-Alkylgruppen substituiert ist, und
- X: für Sauerstoff oder eine Aminogruppe steht, die durch C₁-C₁₀-Alkyl, Benzyl oder Phenyl substituiert ist,
zu diastereomerenreinen Verbindungen der allgemeinen Formeln (Va) und (Vb) 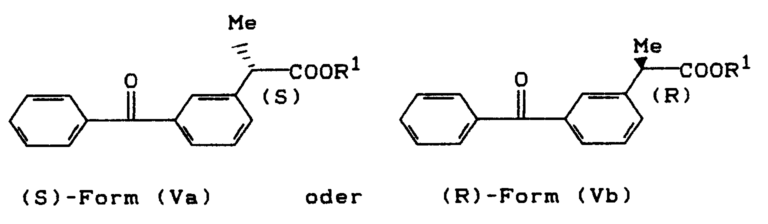
gegebenenfalls in Gegenwart von inerten organischen Lösungsmitteln umsetzt und diese dann nach üblichen Methoden zu enantiomerenreinem Ketoprofen hydrolysiert.

Gegenstand der Erfindung sind auch die diastereomerenreinen Ester der Formel (V), in welcher R¹ die oben angegebene Bedeutung hat und ihre Verwendung bei der Herstellung von (R)- oder (S)-Ketoprofen.

Die Herstellung der erfindungsgemäßen neuen, diastereomerenreinen Verbindungen der allgemeinen Formel (V) erfolgt vorzugsweise aus dem Chlorid der Säure der Formel (II) in Gegenwart eines tertiären Amins, insbesondere eines Alkyl- oder Cycloalkylamins mit jeweils bis zu 10 C-Atomen, insbesondere mit bis zu 6 C-Atomen, eines Benzyl- oder eines Phenylamins, wobei die Phenylringe gegebenenfalls durch Halogen, Alkyl oder Alkoxy mit bis zu 5 C-Atomen substituiert sind,
in einem unpolaren Lösungsmittel bei Temperaturen zwischen -80°C und +25°C mit einem optisch aktiven Alkohol der Formel (IV), in welcher R¹ die oben angegebene Bedeutung hat.

Die Hydrolyse von Verbindungen der allgemeinen Formel (V) zu den enantiomerenreinen Formen des Ketoprofens erfolgt vorzugsweise unter basischen Bedingungen. Der Enantiomerenüberschuß kann durch übliche Kristallisationsverfahren erhöht werden.

Als besonders bevorzugte optisch aktive Alkohole der allgemeinen Formel (IV) seien genannt α-Hydroxyester und α-Hydroxyamide wie Milchsäure-isobutyl-, ethyl-, methyl-, isopropyl- und -benzylester oder N,N-Dimethylmilchsäureamid, sowie α-Hydroxylactone oder α-Hydroxyimide. Ebenso bevorzugt sind die entsprechenden Ester der allgemeinen Formel (V).

Bevorzugte tertiäre Amine für die Umsetzung der Säurechloride zu den Ketenen sind offenkettige oder cyclische Trialkylamine. Besonders bevorzugte Amine sind Tri-(C₁-C₅)-alkylamine wie z.B. Trimethylamin, Dimethylethylamin, Triethylamin, 1,4-Diaza-bicyclo-[2,2,2]-octan (DABCO) oder N-Methylpyrrolidin.

Die Gegenwart des Amins ist für das vorliegende Verfahren nicht kritisch. Bevorzugt wird das tertiäre Amin in einem Verhältnis von 0,01 bis 10 Moläquivalenten bezogen auf Keten (III) eingesetzt. Besonders bevorzugt wird der Einsatz in einem Verhältnis von 0,01 bis 2 Moläquivalenten Amin bezogen auf Keten.

Geeignete Lösungsmittel für die Reaktion sind aprotische Flüssigkeiten, die gegenüber den Reaktionspartnern inert sind. Beispiele für derartige Solventien sind Kohlenwasserstoffe, wie z.B. Hexan, Heptan, Toluol oder Cyclohexan oder Ether wie z.B. Diethylether, Tetrahydrofuran oder tert.-Butylmethylether. Bevorzugte Lösungsmittel sind Hexan, Heptan und Toluol.

Bei dem vorliegenden Verfahren wird der α-Hydroxyester oder das α-Hydroxyamid zu einer Lösung des Ketens gegeben. Bei größeren Ansätzen kann es aber verfahrenstechnisch von Vorteil sein, das Keten (III) in Lösung zum α-Hydroxyester oder α-Hydroxyamid zuzugeben.

Die Herstellung des Ketens erfolgt aus den Säurechloriden mit Hilfe der Basen, in einem unpolaren Lösungsmittel, wobei zweckmäßigerweise das Lösungsmittel verwendet wird, das auch für die Umsetzung des Ketens (III) verwendet wird, bei Temperaturen zwischen -20°C und 50°C, wobei das Keten bei geeigneter Reaktionsführung sofort gebildet wird und auf eine Isolierung verzichtet werden kann. Die gebildeten Aminhydrochloride stören im allgemeinen bei den nachfolgenden Reaktionen nicht.

Das Molzahlverhältnis von Keten (III) zu α-Hydroxyester oder α-Hydroxyamid (IV) kann 1 bis 1,5, vorzugsweise 1 bis 1,2 betragen. Besonders bevorzugt ist ein Molzahlverhältnis von nahezu 1:1. Die bevorzugte Reaktionstemperatur liegt im Bereich von -20°C bis -100°C, besonders bevorzugt ist jedoch eine Temperatur von -78°C bis -60°C. Die einsetzbare Ketenkonzentration liegt in einem Bereich von 1,0 M bis 0,01 M. Die Reaktionsmischung wird bei der bevorzugten Temperatur für 0,5 bis 16 Stunden gerührt. Bei vollständigem Umsatz werden die diastereomeren Ester nach Standardverfahren und Techniken aufgearbeitet und gereinigt, wie z.B. Chromatographie.

Die entsprechenden diastereomeren Ester (V) können in üblicher Weise, z.B. unter sauren Bedingungen (siehe EP 0 179 487 oder R. Lattrell et al., Ann. Chem., 1974, 870) oder unter basischen Bedingungen (D.A. Evans et al., Tetrahedron Lett., 1987, 6141) oder bevorzugt basisch im System LiOH/H₂O/Acetonitril/Heptan/0°C (R.D. Larsen et al., J. Am. Chem. Soc., 111, 7650 (1989)) zu der enantiomerenreinen Carbonsäure Ketoprofen gespalten werden, die mit hohen Enantiomerenüberschüssen anfällt.

### Beispiele

Die Enantiomerenanalytik erfolgte nach bekannten Methoden (z.B. nach EP 379 917) durch chromatographische Trennung an chiralen stationären Phasen.

### Vergleichsbeispiel A

### Herstellung von (2S)-2-(3-Benzoyl-phenyl)-propionsäure-(S)-ethylmilchsäureester durch Addition des (S)-Ethylmilchsäureesters zum Keten (Konventionelles Procedere)

Zu einer Lösung von 440,0 ml (4,02 mol) N-Ethyldimethylamin in 1740 ml wasserfreiem Toluol gibt man innerhalb von 10 Minuten 366,5 g (1,34 mol) (R/S)-2-(3-Benzoylphenyl)-propionsäurechlorid bei Raumtemperatur zu. Nach erfolgter Zugabe konnte mittels IR-Spektroskopie kein (R/S)-2-(3-Benzoyl-phenyl)-propionsäurechlorid mehr nachgewiesen werden. Die erhaltene Mischung wird auf -78°C abgekühlt, und innerhalb von 30 min eine Lösung von 184,0 ml (1,64 mol) (S)-Ethyllactat in 1740 ml wasserfreiem Toluol zugetropft, wobei die Innentemperatur -70°C nicht überschreiten darf. Nach erfolgter Zugabe wird noch 3 Stunden bei -78°C gerührt und danach langsam auf Raumtemperatur erwärmt. Dann gibt man 17,0 ml 3-Dimethylaminopropylamin zu, rührt 30 Min. bei Raumtemperatur, wäscht mit Wasser, trocknet mit Magnesiumsulfat und engt zur Trockne ein. Der Rückstand wird durch Filtration über Kieselgel (Gradient Essigester/Cyclohexan 1/10 -> 1/3 v/v) gereinigt. Nach dem Einengen erhält man ein schwach gelbliches Öl.
Ausbeute: 45,49 g (9,6 % d.Th.)
Diastereomerenverhältnis (SS:RS) lt. GC-Analytik: 7,8:1

### Beispiel 1

### Herstellung von (2S)-2-(3-Benzoyl-phenyl)-propionsäure-(S)-ethylmilchsäureester durch Addition des Ketens zum (S)-Ethylmilchsäureester

Eine Lösung von 118,8 ml (1,10 mol) N-Ethyl-dimethylamin in 200 ml wasserfreiem Toluol tropft man innerhalb von einer Stunde in einer gekühlten Mischkammer unter Rührung zu 99,0 g (0,363 mol) (R/S)-2-(3-Benzoylphenyl)-propionsäurechlorid in 200 ml Toluol bei Raumtemperatur zu. Die dabei gebildete Suspension von Keten und Aminhydrochlorid tropft unter Rührung langsam zu einer Lösung von 49,5 ml (0,44 mol) (S)-Ethyllactat in 500 ml wasserfreiem Toluol und 40 ml (0,37 mol) N-Ethyldimethylamin zu, wobei die Innentemperatur -70°C nicht überschreiten darf (Regelbar durch die zugegebene Ketenmenge). Nach erfolgter Zugabe wird noch 3 Stunden bei -78°C gerührt und danach langsam auf Raumtemperatur erwärmt. Dann gibt man 4,6 ml 3-Dimethylaminopropylamin zu, rührt 30 Min. bei Raumtemperatur, wäscht mit Wasser, trocknet mit Magnesiumsulfat und engt zur Trockne ein. Der Rückstand wird durch Filtration über Kieselgel (Gradient Essigester/Cyclohexan 1/10 -> 1/3 v/v) gereinigt. Nach dem Einengen erhält man ein schwach gelbliches Öl.
Ausbeute: 84,98 g (65,4 % d.Th.)
Diastereomerenverhältnis (SS:RS) lt. GC-Analytik: 9,8:1
- ¹H-NMR:: (CDCl₃, 200 MHz) (S,S)-Diastereoisomer:
δ = 1,15 (t, J = 7 Hz, 3H; -O-CH₂-CH₃), 1,47 (d, J = 7 Ht, 3H; -CH=CH₃), 1,57 (d, J = 7 Hz, 3H; -CH=CH₃), 3,87 )q, J = 7 Hz, 1H; -CH-CH₃), 4,10 (q, J = 7 Hz, 2H; -O-CH₂-CH₃), 5,08 (q, J = 7 Hz, 1H; -CH-CH₃), 7,40 - 7,90 (m, 9H; arom. H)
(R,S)-Diastereoisomer
δ = 1,16 (t, J = 7 Hz, 3H; -O-CH₂-CH₃), 1,48 (d, J = 7 Hz, 3H; -CH-CH₃), 1,58 (d, J = 7 Hz, 3H; -CH-CH₃), 3,89 (q, J = 7 Hz, 1H; -CH-CH₃), 4,10 (q, J = 7 Hz, 2H; -O-CH₂-CH₃), 5,08 (q, J = 7 Hz, 1H; -CH-CH₃), 7,40 - 7,90 (m, 9H; arom. H)
- MS:: (CI, Isobutan) m/e = 355 (M+H), 310 (4 %), 249 (6 %), 236 (6 %), 209 (16 %), 105 (6 %)

### Beispiel 2

### Herstellung von (2S)-2-(3-Benzoyl-phenyl)-propionsäure-(S)-ethylmilchsäureester durch Addition des Ketens zum Lactat (Konzentriertere Reaktionsführung).

Eine Lösung von 591 ml (5,46 mol) N-Ethyl-dimethylamin in 500 ml wasserfreiem Toluol tropft man innerhalb von einer Stunde in einer gekühlten Mischkammer unter Rührung zu 492,47 g (1,81 mol) (R/S)-2-(3-Benzoylphenyl)-propionsäurechlorid in 500 ml Toluol bei Raumtemperatur zu. Die dabei gebildete Suspension von Keten und Aminhydrochlorid tropft unter Rührung langsam zu einer Lösung von 247 ml (2,18 mol) (S)-Ethyllactat in 1000 ml wasserfreiem Toluol und 197 ml (1,82 mol) N-Ethyldimethylamin zu, wobei die Innentemperatur -70°C nicht überschreiten darf (Regelbar durch die zugegebene Ketenmenge). Nach erfolgter Zugabe wird noch 3 Stunden bei -78°C gerührt und danach langsam auf Raumtemperatur erwärmt. Dann gibt man 4,6 ml 3-Dimethylaminopropylamin zum, rührt 30 Min. bei Raumtemperatur, wäscht mit Wasser, trocknet mit Magnesiumsulfat und engt zur Trockne ein. Der Rückstand wird durch Filtration über Kieselgel (Gradient Essigester/Cyclohexan 1/10 -> 1/3 v/v) gereinigt. Nach dem Einengen erhält man ein schwach gelbliches Öl.

Ausbeute: 436,98 g (68,0 % d. Th.)
Diastereomerenverhältnis (SS:RS) lt. GC-Analytik: 9,5:1

### Herstellung von (2S)-2-(3-Benzoyl-phenyl)-propionsäure (Ketoprofen) durch basische Verseifung

Eine Mischung von 436,98 g (1,234 mol) (2S)-2-(3-Benzoyl-phenyl)-propionsäure-(S)-ethylmilchsäureester (Diastereomerenverhältnis (SS:RS) lt. GC-Analytik: 9,5:1) in 237,95 g Lithiumhydroxid, 2200 ml Wasser, 2360 ml Acetonitril und 2360 ml Heptan wird über Nacht unter intensiver Rührung mit einem Flügelrührer behandelt. Danach wird die Acetonitril/Wasser-Phase abgetrennt und eingeengt. Der erhaltene Rückstand wird in Essigester aufgenommen und mit Natriumhydroxid-Lösung extrahiert. Der basische Extrakt wird mit Salzsäure angesäuert und mit Essigester extrahiert, getrocknet und eingeengt.

Ausbeute: 300,91 g (1,185 mol) (96,0 % d.Th.)
Enantiomerenüberschuß: 74,9 % (HPLC)

### Vergleichsbeispiel B

### Herstellung von (2R)-2-(3-Benzoyl-phenyl)-propionsäure-(R)-isobutylmilchsäureester durch Addition des (R)-Isobutylmilchsäureesters zum Keten (konventionelles Procedere)

Zu einer Lösung von 40,0 ml (0,370 mol) N-Ethyldimethylamin in 190 ml wasserfreiem Toluol gibt man innerhalb von einer Stunde 49,83 g (0,183 mol) (R/S)-2-(3-Benzoylphenyl)-propionsäurechlorid bei Raumtemperatur zu. Nach erfolgter Zugabe konnte mittels IR-Spektroskopie kein (R/S)-2-(3-Benzoyl-phenyl)-propionsäurechlorid mehr nachgewiesen werden. Die erhaltene Mischung wird auf -78°C abgekühlt, und innerhalb von 30 min eine Lösung von 33,22 ml (0,220 mol) R-Isobutyllactat in 190 ml wasserfreiem Toluol zugetropft, wobei die Innentemperatur -70°C nicht überschreiten darf. Nach erfolgter Zugabe wird noch 3 Stunden bei -78°C gerührt und danach langsam auf Raumtemperatur erwärmt. Dann gibt man 2,5 ml 3-Dimethylaminopropylamin zu, rührt 30 Min. bei Raumtemperatur, wäscht mit Wasser, trocknet mit Magnesiumsulfat und engt zur Trockne ein. Der Rückstand wird durch Filtration über Kieselgel (Gradient Essigester/Cyclohexan 1/10 -> 1/3 v/v) gereinigt. Nach dem Einengen erhält man ein schwach gelbliches Öl.

Ausbeute: 22,28 g (58,10 mmol) (31,7 % d.Th.)
Diastereomerenverhältnis (RR:SR) lt. GC-Analytik: 4,0:1

### Beispiel 3

### Herstellung von (2R)-2-(3-Benzoyl-phenyl)-propionsäure-(R)-isobutylmilchsäureester durch Addition des Ketens zum (R)-Isobutylmilchsäureester

Eine Lösung von 150 ml (1,10 mol) N-Ethyl-dimethylamin in 240 ml wasserfreiem Toluol tropft man innerhalb von einer Stunde in einer gekühlten Mischkammer unter Rührung zu 125,0 g (0,459 mol) (R/S)-2-(3-Benzoylphenyl)-propionsäurechlorid in 240 ml Toluol bei Raumtemperatur zu. Die dabei gebildete Suspension von Keten und Aminhydrochlorid tropft unter Rührung langsam zu einer Lösung von 50,0 ml (0,44 mol) (R)-Isobutyllactat in 475 ml wasserfreiem Toluol und 150 ml (0,37 mol) N-Ethyldimethylamin zu, wobei die Innentemperatur -70°C nicht überschreiten darf (Regelbar durch die zugegebene Ketenmenge). Nach erfolgter Zugabe wird noch 3 Stunden bei -78°C gerührt und danach langsam auf Raumtemperatur erwärmt. Dann gibt man 4,6 ml 3-Dimethylaminopropylamin zu, rührt 30 Min. bei Raumtemperatur, wäscht mit Wasser, trocknet mit Magnesiumsulfat und engt zur Trockne ein. Der Rückstand wird durch Filtration über Kieselgel (Gradient Essigester/Cyclohexan 1/10 -> 1/3 v/v) gereinigt. Nach dem Einengen erhält man ein schwach gelbliches Öl.

Ausbeute: 113,77 g (0,30 mol) (65,4 % d.Th.)
Diastereomerenverhältnis (RR:SR) lt. GC-Analytik: 9,8:1
- ¹H-NMR:: (CDCl₃, 200 MHz) (S,S)-Diastereoisomer: δ = 0,85 (d, J = 7 Hz, 6H; -CH-(CH₃)₂), 1,49 (d, J = 7 Hz, 3H; -CH-CH₃), 1,57 (d, J = 7 Hz, 3H; -CH-CH₃), 1,83 (mc, 1H; -CH₂-CH-(CH₃)₂), 3,85 (d, 2H, 7 Hz; -CH₂-CH-), 3,89 (q, J = 7 Hz, 1H; -CH-CH₃), 5,11 (q, J = 7 Hz, 1H; -CH-CH₃), 7,40 - 7,90 (m, 9H; arom. H)
(S,R)-Diastereoisomer:
δ = 0,92 (d, J = 7 Hz, 6H; -CH-(CH₃)₂), 1,46 (d, J = 7 Hz, 3H; -CH-CH₃), 1,54 (d, J = 7 Hz, 3H; -CH-CH₃), 1,83 (mc, 1H; -CH₂-CH-(CH₃)₂), 3,85 (d, 2H, 7 Hz; -CH₂-CH-), 3,89 (q, J = 7 Hz, 1H; -CH-CH₃), 5,11 (q, J = 7 Hz, 1H; -CH-CH₃), 7,40 - 7,90 (m, 9H; arom. H)
- MS:: (CI, Isobutan) m/e = 383 (100 %, M+H), 3,27 (14 %), 209 (20 %), 105 (8 %)

### Herstellung von (2R)-2-(3-Benzoyl-phenyl)-propionsäure (Ketoprofen) durch basische Verseifung

Eine Mischung von 113,77 g (0,297 mol) (2R)-2-(3-Benzoyl-phenyl)-propionsäure-(R)-isobutylmilchsäureester (Diastereomerenverhältnis (RR:SR) lt. GC-Analytik: 9,8:1) in 57,49 g Lithiumhydroxid, 525 ml Wasser, 570 ml Acetonitril und 570 ml Heptan wird über Nacht unter intensiver Rührung mit einem Flügelrührer behandelt. Danach wird die Acetonitril/Wasser-Phase abgetrennt und eingeengt. Der erhaltene Rückstand wird in Essigester aufgenommen und mit Natriumhydroxid-Lösung extrahiert. Der basische Extrakt wird mit Salzsäure angesäuert und mit Essigester extrahiert, getrocknet und eingeengt.

Ausbeute: 67,14 g (0,264 mol) (89,0 % d.Th.)
Enantiomerenüberschuß: 74,0 % (HPLC)

## Patentansprüche

1. Diastereomerenreine Verbindungen der allgemeinen Formeln (Va) und (Vb) 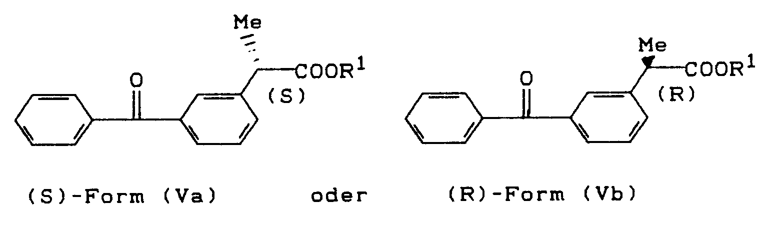 in welcher
R¹ für den Rest 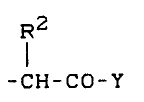 steht, wobei
R² für einen C₁₋₁₀-Alkylrest, C₃₋₅-Cycloalkylrest, Benzyl oder Phenyl steht, und
Y für einen C₁₋₁₀-Alkoxyrest, einen C₃₋₁₀-Cycloalkyloxyrest, Benzyloxy oder Phenoxy steht,
oder
Y für einen Aminrest steht, der durch Alkyl oder Cycloalkyl mit bis zu 10 C-Atomen, Benzyl oder Phenyl gleich oder verschieden substituiert ist,
oder
R¹ für einen Rest des optisch aktiven α-Hydroxylactons oder oder eines cyclischen Amids gemäß der Formel 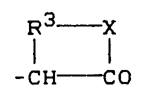 steht, worin
R³ für einen C₂₋₄-Alkylenrest steht, der gegebenenfalls durch 1 oder 2 C₁₋₁₀-Alkylgruppen substituiert ist, und
X für Sauerstoff oder eine Aminogruppe steht, die durch C₁-C₁₀-Alkyl, Benzyl oder Phenyl substituiert ist.

2. Verfahren zur Herstellung von Verbindungen der allgemeinen Formel (V) gemäß Anspruch 1, dadurch gekennzeichnet, daß man racemisches Ketoprofen der Formel (II) 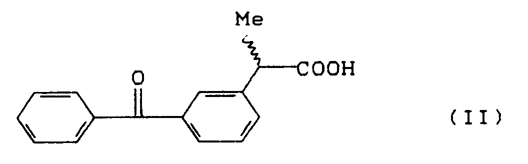 über das Säurechlorid in Anwesenheit von tertiären Aminen zu Ketenen der allgemeinen Formel (III) 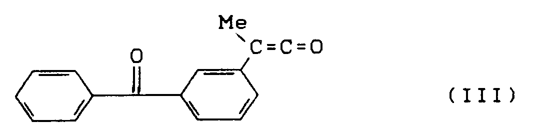 umsetzt, dieses Keten dann in ein separates Reaktionsgefäß überführt und dort bei Temperaturen zwischen -100°C und +25°C mit Alkoholen der allgemeinen Formel (IV)
R¹-OH (IV)
in welcher
R¹ die in Anspruch 1 angegebene Bedeutung hat,
zu diastereomerenreinen Verbindungen der allgemeinen Formeln (Va) und (Vb) 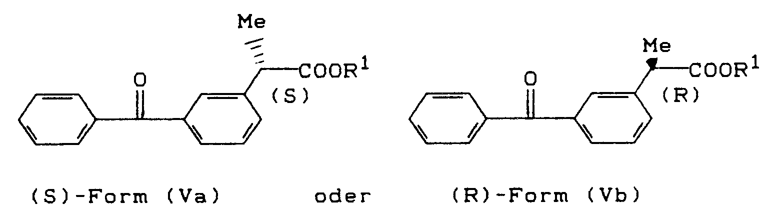 gegebenenfalls in Gegenwart von inerten organischen Lösungsmitteln umsetzt und diese dann nach üblichen Methoden zu enantiomerenreinem Ketoprofen hydrolysiert.

3. Verwendung von Verbindungen der allgemeinen Formel (V) gemäß Anspruch 1 zur Herstellung von (R)- oder (S)-Ketoprofen.

4. Verfahren zur Herstellung von (R)- und (S)-Ketoprofen, dadurch gekennzeichnet, daß man diastereomerenreine Ester der allgemeinen Formel (V) gemäß Anspruch 1 nach üblichen Methoden hydrolysiert.

5. Verfahren gemäß Anspruch 4, dadurch gekennzeichnet, daß man die Hydrolyse unter basischen Bedingungen durchführt.
